# EUROPEAN PATENT APPLICATION

(11) **EP 4 685 474 A1**
(43) Date of publication of application: **28.01.2026**
(21) Application number: 25192292.8
(22) Date of filing: 28.07.2025
(51) Int. Cl.: G01N 27/414

(54) **BIOSENSING DEVICE BASED ON FIELD-EFFECT TRANSISTOR WITH NOISE REDUCTION METHOD**

(30) Priority: 26.07.2024 PT 2024119622
(71) Applicant: EXOTICTARGET - LDA, 4900-425 Viana do Castelo (PT)
(72) Inventor: DO CARMO DE SEQUEIRA ANTUNES, MARIA BEATRIZ, 7350-507 VILA BOIM (PT); TEIXEIRA DUARTE FERREIRA, HUGO ALEXANDRE, 2795-049 LINDA-A-VELHA (PT); VIEIRA MARUJO, NUNO TOMÁS, 4900-425 VIANA DO CASTELO (PT)
(74) Representative: Patentree

(57) **Abstract**

The present disclosure relates to a highly sensitive biosensing device based on field-effect transistors (FETs) for the detection and quantification of pH and biomarkers in urine samples. The described biosensing device has applications in medical diagnostics, environmental monitoring, and personalized health management, offering a robust tool for early detection and monitoring of various health conditions.

## Description

### TECHNICAL FIELD

The present disclosure relates to a biosensing device utilizing a Field-Effect Transistor (FET) to detect biomarkers in urine samples. The device incorporates an advanced noise reduction method to enhance the signal-to-noise ratio (SNR) and detection accuracy. This device is particularly effective in measuring pH and detecting various analytes, such as electrolytes, metabolites, proteins, nucleic acids, lipids, carbohydrates and other biomolecules, by responding to changes in the electrical properties of the transistor's gate.

### BACKGROUND

Early detection and monitoring of health conditions are critical in modern medicine. Current diagnostic methods often require expensive, time-consuming, and invasive procedures. Urine analysis offers a non-invasive alternative for diagnosing and monitoring various health conditions, as it contains valuable biomarkers. A need exists for a robust, sensitive, and cost-effective mean capable of detecting and quantifying biomarkers and pH variations in urine samples.

Urine is a promising biological fluid for diagnostics due its unique characteristics, such as a painless and non-invasive collection, abundance of biomarkers, and the ability to detect diseases early, enabling for timely intervention and improved patient outcomes [1]. Maintaining the balance of urine pH and urine metabolite concentrations is vital for good health, with abnormal levels indicating various conditions such as urinary tract infection, kidney failure, or metabolic disorders.

Urinalysis is commonly performed using traditional methods such as urine culture, urine microscopy, urine biochemistry and dipsticks tests. While the former three are staples in clinical settings for assessing urinary tract health and diagnosing various conditions, they can be expensive, complex, and time-consuming [2]. Regarding dipstick tests, despite their widespread use due to ease of use, quick response time, and low cost, interpreting results with the naked eye can be subjective, further depending on variations in lighting and/or individual visual perception which affect color interpretation, leading to inaccuracies [3].

WO2018122403A1 discloses a urine analyzer device, configured to be installed in a toilet bowl, to be used several times. The device comprising a holder member to attach the device to the rim of the toilet bowl, an electronic unit, enclosed in a housing, a urine reception area, with at least one electro-chemical sensor, configured to measure a quantity of at least one substance contained in urine, such as a physiological compound or chemical component, a wireless coupler to send resulting data to a remote computing device, wherein the electro-chemical sensor comprises at least a ion selective Field Effect Transistor (ISFET), configured to sense levels of one or more physiological ions, thereby providing a urine analyzer device, readily available at home, simply installed in a toilet bowl, and that can be used several times subsequently.

WO1995018373A1 discloses a urine analyzing unit of the type mounted on a closet, that can be readily mounted to a standard type water closet so as to sample and analyze urine in a toilet. The unit comprises a substantially self-contained housing which is mounted to the upper surface of the standard type water closet, between a bowl and a flush water supply section. Pivotally mounted to the housing is a seat, and mounted to the seat is a urine sampling device, by which urine excreted into the bowl is sampled in the air within a space of the bowl. Within the housing are a urine analyzing unit provided with a polarograph flow cell and a syringe pump for transferring a urine sample to the flow cell together with a carrier liquid. The polarograph flow cell has an active electrode which carries an enzyme. In one embodiment, a tank for the carrier liquid is received in the housing. In another embodiment, a tank for the carrier liquid is mounted to a closet cover. Also, a method of reconstructing a toilet provided with a standard type closet into a toilet having a urine analyzing function is provided, and a prefabricated type seat assembly having the urine sampling device incorporated beforehand into the seat is used.

EP0687909B1 discloses a method of sampling urine in a toilet provided with a standard type water closet, and an apparatus therefore. An elongated urine taking vessel extending transversely of the closet is used. The urine taking vessel is taken to an optimum urine taking position within a bowl space, along an inner surface of bowl of the closet, while being supported such that an axis of the urine taking vessel is extended perpendicular to a vertical, central surface of the closet and horizontally. As the urine taking vessel can accommodate a deviation of an individual urination in a longitudinal direction and in a right and left direction, it is possible to efficiently take urine with the urine taking vessel of a small size. The urine taking vessel is mounted to an end of a swing arm having a horizontal axis of rotation. In a simplest embodiment, the swing arm is given only a rotating movement. In a more sophisticated embodiment, the urine taking vessel is taken to a urine taking position by the swing arm performing rotating and translating movements. After use, the urine taking vessel is received in a washing chamber, which is substantially concealed by a front portion of a seat, and undergoes washing by pressure water jetted from a jet nozzle. In a preferred embodiment, a urine sampling apparatus is mounted to the seat.

EP0473072B1 discloses a toilet system with urine assay function, which comprises a toilet stool having a toilet bowl and a small urine-sampling cavity. A frame is mounted on the toilet stool to support a testing sheet handling and transfer mechanism, which comprises a swingable carriage and a slidable arm supported by the carriage for linear telescoping movement. The carriage is swingable about a vertical axis between a first position, in which the carriage is oriented toward the sampling cavity, and a second position, in which the carriage is oriented to a lateral side of the stool wherein a disposal station is provided. The toilet system enables the use of testing sheets which are made from water insoluble materials. Various other embodiments are also disclosed. The system provides a high degree of reliability of analysis.

These facts are described in order to illustrate the technical problem solved by the embodiments of the present document.

### GENERAL DESCRIPTION

The present description relates to a highly sensitive biosensing device utilizing a Field-Effect Transistor (FET) to detect and quantify biomarkers and pH in urine samples. The device may incorporate an advanced noise reduction method that combines classical signal processing techniques with machine learning methods, thereby enhancing the signal-to-noise ratio (SNR) and improving detection accuracy.

The device employs a microfabricated gold electrode functioning as an extended-gate (EG) and incorporates advanced signal processing techniques to enhance measurement accuracy and reliability.

In some embodiments, the disclosure pertains to a multiplexed biosensor capable of simultaneous detection of multiple urinary biomarkers, providing comprehensive health insights. The biosensor is equipped with a regenerating means that allows for the reuse of the sensor surface, thus extending the operational lifespan and maintaining consistent performance.

The biofunctionalization method described in the disclosure involves the preparation of the extended-gate electrode surface using self-assembled monolayers (SAMs) and specific binding agents such as antibodies, ensuring selective and sensitive detection of target biomarkers.

Additionally, the disclosure may include a noise reduction method that may comprise classical signal processing techniques and machine learning methods to improve the signal-to-noise ratio (SNR). This method adapts to varying noise levels and patterns, ensuring accurate detection even in the presence of significant background noise.

The calibration methods for both pH measurement and biomarker detection are also disclosed, providing protocols for establishing accurate calibration curves essential for quantitative analysis.

Also, the device may integrate a microelectronic platform comprising a metal-oxide-semiconductor field-effect transistor (MOSFET) and a readout circuit. This platform facilitates the amplification and processing of signals, enabling real-time or near real-time operation of the biosensor.

The described biosensing device has potential applications in medical diagnostics, environmental monitoring, and personalized health management, offering a robust tool for early detection and monitoring of various health conditions.

In an embodiment, the disclosed biosensing device comprises a microfabricated gold electrode as an extended gate (EG) for the FET, and a detection surface. The device demonstrates a robust response as a pH sensor, with a sensitivity of 2.20 µA/pH. For biomarker detection, the device successfully detects IgG, among other biomarkers, with antigen-antibody binding causing a measurable change of approximately 4 µA in its response.

In another aspect of the present disclosure, the microfabrication of the detection surface involves several lithography steps:
a first lithography step, where a positive photoresist is deposited and exposed to define the test electrodes, more specifically, A thin film of Cromium/Gold (Cr/Au) is deposited, followed by lift-off to create the required pattern, preferably the thickness of Cr is from 2 nm to 50 nm, more preferably from 3 nm to 10 nm, more preferably 5 nm, and the thickness of the Au is from 100 nm to 300 nm, preferably from 150 nm to 250 nm, more preferably 200 nm;
a second lithography step, where similar steps are repeated to define the reference electrodes with a thin film of Titanium/Platinum (Ti/Pt), preferably the thickness of Ti is from 2 nm to 50 nm, more preferably from 3 nm to 10 nm, more preferably 5 nm, and the thickness of the Pt is from 200 nm to 400 nm, preferably from 250 nm to 350 nm, more preferably 300 nm; and
a final lithography step, where the sample is passivated with Silicon Nitride (Si₃N₄), and contact vias are opened to expose the pads for biofunctionalization.

In an embodiment, the biofunctionalization and noise reduction methods provide enhanced sensitivity and specificity in detecting targeted biomarkers in urine samples.

In an embodiment, a functionalization process is applied to the detection surface comprising:
a SAM Formation, where the EG is incubated in a solution of MUA and MUD to form a self-assembled monolayer; and/or
an activation and binding, where carboxyl groups are activated using EDC/NHS, followed by incubation with protein A and blocking with BSA.

In an embodiment, the microelectronic circuit comprises:
a transducer element, more specifically a MOSFET coupled to a readout circuit with an operational amplifier, to amplify the drain-source current (I_{ds}); and
a signal processing element, namely a data processor that processes the transduced signals using developed algorithms, preferably said data processor being an Arduino.

In an embodiment, for the pH sensing, the sensor's I-V characteristics are measured at different pH values, showing a linear response with a sensitivity of 2.20 µA/pH.

In an embodiment, for the IgG detection, a sandwich immunoassay format is used. The functionalized EG is exposed to IgG, and specific binding induces measurable changes in the drain-source current (I_{ds}), correlating with IgG concentration.

It is also disclosed a method for denoising comprising ensemble techniques to enhance the robustness and reliability of the noise reduction method, ensuring cleaner signals for biomolecular detection. It is also disclosed an advanced noise reduction method comprising classical signal processing techniques (PCA, DWT, Kalman Filter) and machine learning methods (RNN, LSTM, adaptive filtering) to improve the signal-to-noise ratio (SNR) and detection accuracy of the biosensor device.

In an embodiment, the noise reduction method can be selected from one or more of the following methods:
Data Acquisition and Preprocessing, with high-frequency data acquisition and low-pass filtering to remove high-frequency noise;
Principal Component Analysis (PCA), for dimensionality reduction and identification of principal components;
Discrete Wavelet Transform (DWT), for decomposition of the signal to isolate noise components;
Kalman Filter, to estimate the true signal from noisy observations;
Recurrent Neural Networks (RNN) that utilize Long-Short Term Memory (LSTM) networks to model and predict signal dynamics;
Adaptive Filtering for the continuous adjustment of filter coefficients using Least Mean Squares (LMS) and Recursive Least Squares (RLS) methods; and
Ensemble Methods that combining multiple denoising methods for robust noise reduction; or a combination of these.

In an embodiment, the noise reduction method comprises an adaptive filtering to minimize noise and one or more machine-learning models to dynamically respond to new noise patterns; and enhancing signal-to-noise ratio (SNR) and detection accuracy for specific biomarkers.

It is also disclosed, a biosensor device comprising a FET for detecting pH and biomarkers in urine samples (e.g., urine metabolites), utilizing a microfabricated gold electrode as an extended gate (EG).

In an embodiment, the biosensor system also comprises a microfabricated gold electrode functioning as an extended-gate (EG); and a readout circuit for recording and amplifying signals resulting from modifications in the gate's electrical properties.

In an embodiment, the biosensor is designed for multiplexed detection, enabling simultaneous measurement of multiple urinary biomarkers. Herein, the extended gates may have a range of contact surface areas for the detection of various levels of each of the multiple urinary biomarkers.

It is disclosed that the biosensor further comprises a regeneration mechanism allowing for the cleaning and reuse of the sensor surface using Glycine-HCl, or similar solutions. In an embodiment, the regeneration method comprises the steps of adding Glycine-HCl to the device after IgG testing, to return the current value to the initial baseline without IgG; and of ensuring the sensor surface can be regenerated multiple times without compromising functionality.

It is also disclosed, a method for microfabricating the detection surface, including the deposition of Cr/Au and Ti/Pt thin films, passivation with Si₃N₄, and multi-step lithography to create and expose the necessary electrode pads for subsequent biofunctionalization. Furthermore, it is also disclosed, a multi-step biofunctionalization method involving SAM formation, carboxyl group activation, protein A binding, and blocking with BSA to prepare the EG for specific biomarker detection.

In an embodiment, the method for biofunctionalizing the extended-gate electrode of the FET biosensor comprises: cleaning the chip with deionized water and ethanol, followed by ultraviolet (UV) light treatment; incubating the gold pads in a solution of mercaptoundecanoic acid (MUA) and 11-mercapto-1-undecanol (MUD) to form a mixed self-assembled monolayer (SAM); activating the carboxyl groups of the SAM using a solution of N-ethyl-N'-(3-(dimethylamino)propyl) carbodiimide (EDC) and N-hydroxysuccinimide (NHS); incubating the electrode with anti-immunoglobulin G (Anti-IgG); blocking unbound sites with a bovine serum albumin (BSA) solution.

It is also disclosed, a microelectronic readout circuit comprising a MOSFET transducer, an operational amplifier to amplify drain-source current (I_{ds}), and an data processor for signal processing using any of the previously disclosed methods, in particular, said data processor being an Arduino.

It is also disclosed, a method for measuring and calibrating the pH sensitivity of the biosensor using standard buffer solutions and detecting IgG using a sandwich immunoassay format, correlating changes in drain-source current (I_{ds}) with biomarker concentration.

In an embodiment, the method for determining the pH sensitivity of the FET biosensor comprises the steps of:
delivering solutions of different pH values to the pads using a micropipette; recording the current-voltage (I_{ds}-V_{ref}) curves for each solution;
selecting a gate voltage value within the linear operating region of the MOSFET; and
recording the respective I_{ds} value for each pH value to determine the calibration curve (I_{ds}-pH).

In an embodiment, the method for detecting and calibrating biomarkers using the disclosed device comprises:
applying phosphate-buffered saline (PBS) solutions spiked with various concentrations of immunoglobulin G (IgG) to the pads;
recording the current-voltage curve (I_{ds}-V_{ref}) for each solution; and
assessing the limit of detection (LOD), linear range, and sensitivity of the device.

In an embodiment, the biosensing device comprises a microelectronic circuit which comprises: a sensing surface (chip) coupled with a MOSFET; a readout circuit comprising an operational amplifier to amplify the drain-source current (I_{ds}) and an data processor for processing the transducer signals using custom algorithms, in particular, said data processor being an Arduino.

The present disclosure relates to a biosensor device for detecting pH and biomarkers in urine samples, comprising a field-effect transistor (FET) comprising a microfabricated gold electrode as an extended gate (EG), as described herewith.

In an embodiment, the EG of the biosensor device is microfabricated using a process comprising: depositing a positive photoresist and exposing it to define test electrodes; depositing a thin film of Cr/Au (5 nm/200 nm); performing lift-off to create a pattern; repeating the above steps for reference electrodes with a thin film of Ti/Pt (5 nm/300 nm); passivating the sample with Si₃N₄; opening contact vias to expose pads for biofunctionalization.

In an embodiment, the surface functionalization of the EG comprises: forming a self-assembled monolayer (SAM) by incubating in a solution of MUA and MUD; activating carboxyl groups using EDC/NHS; incubating with protein A; blocking with BSA.

An embodiment may comprise a method for measuring pH sensitivity using the biosensor device, comprising: calibrating the sensor with standard buffer solutions; measuring I_{ds}-V characteristics at different pH values; and recording changes in I_{ds} to determine sensitivity.

It is also disclosed, a method for detecting IgG using the biosensor device, comprising: using a sandwich immunoassay format; exposing the functionalized EG to IgG in PBS; rinsing and incubating with a secondary antibody conjugated with HRP; and measuring changes in I_{ds}.

An embodiment may comprise a noise reduction method for use with the biosensor device, comprising one or more of the following:
data acquisition and preprocessing involving high-frequency data acquisition and low-pass filtering;
applying PCA for dimensionality reduction; using DWT to decompose the signal;
employing a Kalman Filter to estimate the true signal from noisy observations;
utilizing RNN with LSTM networks to model signal dynamics;
implementing adaptive filtering using Least Mean Squares (LMS) and Recursive Least Squares (RLS) methods; and
combining multiple denoising methods using ensemble techniques.

An aspect of the present disclosure relates to a biosensing device for measuring at least one biomarker of a human biological sample, comprising a channel for allowing the flow of the human biological sample, an inlet at one end of said channel and an outlet the other end of said channel, wherein the said channel comprising:
a sensor for transducing a signal of the at least one biomarker of the biological sample;
an amplifier electrically connected to the sensor for amplifying the transduced signal; and a computer processor configured to:
   acquire the amplified signal, and
   quantify the at least one biomarker in the biological sample;
wherein the sensor comprises one or more field-effect transistors, FET;
wherein each of the one or more the field effect transistor, FET, comprises an extended gate; and
wherein each of the one or more extended gates comprises a plurality of analyte-binding elements for detecting the at least one biomarker.

In an embodiment, the biosensing device comprises a toilet or a urinal, wherein the inlet is connected to the toilet, or to the urinal for receiving the human biological sample, and wherein the outlet is connected to a sewer or to a sterilized container.

In an embodiment, the human biological sample is at least one of urine, blood, saliva, sweat and feces, in particular, the human biological sample is urine.

In an embodiment, the biosensing device comprises a glass or ceramic surface, wherein the sensor is arranged on the glass or ceramic surface, and wherein:
said glass or ceramic surface is suitable to be sterilized;
the one or more extended gates are placed onto said glass or ceramic surface; and
wherein each of the one or more extended gates comprises:
   a chromium layer deposited onto said glass or ceramic surface; and
   a gold layer placed deposited on the chromium layer, on which are arranged the plurality of analyte-binding elements.

In an embodiment, the biosensing device comprises a direct-current voltage source, wherein the sensor comprises one or two reference electrodes [i.e., reference pads], wherein said reference electrodes are electrically connected to the direct-current voltage source, and wherein each of the one or two reference electrodes comprises:
a titanium layer; and
a platinum layer deposited on the titanium layer, for electrically connecting to the direct current voltage source.

In an embodiment, the titanium layer of at least one of the one or two reference electrodes is deposited on the gold layer, and wherein the gold layer is deposited onto the chromium layer, and further wherein the chromium layer is deposited onto the glass or ceramic surface [typical thicknesses of the layers are as follows: electrodes with 5 nm of chromium and 200 nm of gold; electrodes with 5 nm of titanium and 300 nm of platinum].

In an embodiment, the direct-current voltage source is from 0.2 V to 2 V; more preferably from 0.4 V to 0.8 V.

In an embodiment, the sensor further comprises a multiplexer for multiplexing each current signal from each of the one or more field-effect transistors, wherein each of the two or more field-effect transistors is electrically connected to the multiplexer.

In an embodiment, each of the one or more extended gates has a length ranging from 0.1 mm to 10 mm; preferably from 0.5 mm to 5 mm; and/or wherein the extended gate has an aspect ratio of width per length ranging from 1:100 to 1:1; preferably from 1:10 to 1:1.

In an embodiment, acquiring the amplified signal comprises a step of removing a noise component from said demultiplexed signal.

In an embodiment, the biosensing device comprises revolving assembly of a plurality of sensors, wherein each sensor of the plurality of sensors comprises one or more field-effect transistors, FET, wherein each of the one or more the field effect transistor, FET, comprises an extended gate, and wherein each of the one or more extended gates comprises a plurality of analyte-binding elements for detecting the at least one biomarker.

In an embodiment, the biosensing method comprises the following steps for obtaining the one or more extended gates and the one or two reference electrodes:
providing a glass surface;
coating the glass surface with a layer of a first photoresistor material;
patterning onto the first photoresistor layer by laser direct-writing;
depositing a chromium layer and a gold layer onto the glass surface and the patterned first photoresistor layer;
removing the first photoresistor layer to obtain the glass surface with patterned chromium and gold layers;
coating with a layer of a second photoresistor material;
patterning onto the second photoresistor layer by laser direct-writing;
depositing a titanium layer and a platinum layer onto the onto the second photoresist and the patterned chromium and gold layers;
removing the second photoresistor layer to obtain patterned titanium and platinum layers.

In an embodiment, the biosensing method comprises the steps of:
incubating the one or more extended gates with an ethanolic solution of mercaptoundecanoic acid, MUA, and 11-mercapto-1-undecanol, MUD, with a molar proportion ranging from 1:3 to 2:3 (%mol._{MUA}/%mol._{MUD}), for the formation of a mixed self-assembly monolayer (SAM);
incubating the extended gate with a solution of the analyte-binding elements;
incubating the extended gate with a bovine serum albumin, BSA, solution, for blocking unbound parts of the extended gate.

In an embodiment, the incubation temperature of the extended gate with the ethanolic solution of mercaptoundecanoic acid, MUA, and 11-mercapto-1-undecanol, MUD, ranges from about 20 °C to 25 °C.

In an embodiment, the incubation temperature of the solution of analyte-binding elements is from 2 °C to 8 °C; preferably from about 3 °C to 6 °C.

In an embodiment, the incubation temperature of the bovine serum albumin, BSA, solution ranges from about 20 °C to 25 °C.

In an embodiment, the pH of the bovine serum albumin, BSA, solution ranges from 7 to 7.8; preferably from 7.2 to 7.6.

In an embodiment for measuring biomarkers of a biological sample, comprises the steps:
feeding the biosensing device according to any of the disclosed embodiments with a biological sample; and
adding a Glycine-HCl regeneration flow to the one or more extended gates, after operation of the sensor, in particular after each operation of the sensor.

In an embodiment, said biomarkers includes a pH biomarker.

### BRIEF DESCRIPTION OF THE FIGURES

For an easier understanding, figures are herein attached, which represent preferred embodiments that do not intend to limit the object of the present description.
**Figure 1****:** Schematic representation of an embodiment of the chip to scale.
**Figure 2****:** Representation of a sketch of the microfabrication method of the chip.
**Figure 3A****:** Schematic diagram of the microelectronic circuit for the Bio-FET device.
**Figure 3B****:** Breadboard assembly of said microelectronic circuit.
**Figure 3C****:** Schematic circuit of an embodiment for processing signals from pads of different sizes.
**Figure 4****:** Schematic diagram of IgG immobilization method on an embodiment with the chip surface.
**Figure 5****:** Plot representing I_{ds} current over time after adding HCl and NaOH to a pH 7 solution.
**Figure 6A****:** Plot representing drain-source current-voltage (I_{ds}-V_{ref}) curves for the six solutions tested in exemplary embodiments, wherein part of the curves in the linear operating region of the FET are represented in the inset plot.
**Figure 6B****:** Calibration curve from said exemplary embodiments.
**Figure 7****:** Estimated layer thickness of the extended gates of some embodiments, obtained by ellipsometry.
**Figure 8****:** Plot representing I_{ds} current over time after adding IgG and Glycine-HCl to PBS.
**Figure 9****:** Plot representing drain-source current-voltage (I_{ds}-V_{ref}) curves for the four PBS-IgG solutions tested in exemplary embodiments, wherein part of the curves in the linear operating region of the FET are represented in the inset plot.
**Figure 10****:** Schematic flow diagram of an embodiment of the method for the regeneration.
**Figure 11****:** Plot of a real-time response of I_{ds} values during cyclic exposures to buffer solution (PBS) and creatinine (Cre), interspersed with sensor surface regeneration steps.
**Figure 12****:** Bar plot of Average I_{ds} values for different detection cycles, comparing the sensor response to the buffer solution (PBS) and creatinine.
**Figure 13****:** Schematic illustration of the structure and operation of an embodiment of the biosensing device.

### DETAILED DESCRIPTION

The present disclosure relates to a biosensing device utilizing a Field-Effect Transistor (FET) to detect biomarkers in urine samples.

In an embodiment, the FET functions as the transducer element in the biosensing device. In a typical FET-based biosensor, the gate of the transistor is modified with a bioreceptor layer (e.g., antibodies, enzymes, or aptamers) that is specific to the target biomarkers in a liquid sample, e.g., urine. When urine containing the target biomarker is introduced to the biosensor, the biomarkers interact with the bioreceptor layer on the gate, binding to each other. This binding event triggers a change in the electrical properties of the FET's gate, which modulates the current flowing through the transistor's channel. The change in current is then processed and interpreted to provide quantitative information about the detected analyte.

Advantageously, the FETs are particularly suitable to detect biomarkers in urine samples due to their high sensitivity, label-free detection, rapid response time, and ability to be miniaturized. They also offer low power consumption, compatibility with various bioreceptors, and scalability for cost-effective mass production.

In an embodiment, the biosensing device can detect a variety of biomarkers in urine samples, including electrolytes, metabolites, peptides and proteins (including enzymes and immunoglobulins), lipids, carbohydrates, nucleic acids and others, such as hormones, viral particles and cells.

In an embodiment, the biosensing device is particularly arranged to identify multiple biomarkers associated with various diseases and conditions. The device features multiplexing capability, allowing it to detect a wide range of biomarkers from a single sample. This includes biomarkers related to cancer, such as those for breast, prostate, and colorectal cancers, as well as markers for kidney diseases, urinary tract infections and other metabolic, endocrine, immune-mediated, cardiovascular neurological and psychiatric disorders.

For better results, an embodiment further comprises an advanced noise reduction method by significantly increasing signal clarity. It effectively filters out background noise, ensuring that the biomarker signal is more prominent and accurate. This leads to improved sensitivity, allowing the device to detect lower concentrations of biomarkers that might otherwise be obscured by noise.

An embodiment may comprise a higher SNR improves sensitivity, allowing for the detection of lower concentrations of biomarkers, which may aid in early diagnosis and in monitoring of low levels of biomarkers. Furthermore, an embodiment may increase accuracy by reducing the likelihood of false positives and false negatives, leading to more reliable diagnostic results. Additionally, enhancing SNR may help to distinguish the target biomarker from other substances or noise in the sample, provided a suitable bioreceptor layer. Overall, a higher SNR of an embodiment may contribute to clearer, more accurate, and consistent measurements, that enhance the performance of the biosensing device.

In an embodiment, the biosensing device can be used for other types of samples than urine, such as blood, saliva, sweat, and feces. To function with different types of samples, the biosensing device may require certain modifications, such as tailoring the bioreceptor layer to the specific biomarkers present in the sample type, implementing specific preparation steps to handle different sample matrices (such as dilution, filtration, or centrifugation to remove interfering substances), and adjusting surface chemistry to ensure optimal binding and stability of the bioreceptor layer in different biological environments. Additionally, calibrating the sensor for the specific concentration ranges and sensitivities required for the new sample type and incorporating methods to mitigate interference from other components in the sample that could affect the sensor's accuracy and reliability are also necessary.

In an embodiment, the detection may comprise at least one machine-learning model configured to find complex, and nonlinear (e.g., short-, medium-, and/or long-range correlations) signatures in the biosensor signals which are related to noise or otherwise to biomolecular recognition. Therefore, this method may enable the reduction of noise from signals, improving SNR and consequently sensitivity and accuracy of detection. This method can be suitable in dynamic or varying noise conditions.

The main challenges addressed in an exemplary embodiment by combining classical signal processing techniques with machine learning methods might include normalization of signal inputs, higher computational demands, and eventual limitations for having a real-time output if desirable, and the explainability of non-linear machine-learning methods, and therefore the present disclosure may require proper testing with synthetic and controlled signals to support interpretation.

The present disclosure may be particular by implementing noise reduction methods, a higher sensitivity, and therefore higher accuracy is achieved.

In an embodiment, the device may be calibrated using, on one end, surrogate signals and, on the other hand, urine samples spiked with the biomarkers of interest, with known concentrations, and finally with a confrontation of real urine samples testing using gold-standard laboratory techniques and instruments. The procedures and protocols followed during the calibration process are not different from best practices, that use samples with known concentrations and/or confronted against gold-standard laboratory techniques and instruments. Nonetheless, herein calibration curves that be derived from a dictionary variable and from a machine learning model, may be used in separate or in combination.

The potential applications of the disclosed biosensing device in medical diagnostics might be extensive and impactful, as it can facilitate early disease detection by identifying biomarkers associated with conditions such as cancer, diabetes, and infections at their initial stages, which may improve treatment outcomes. The disclosed device may also be useful for monitoring chronic conditions like diabetes and cardiovascular diseases, allowing for timely adjustments in treatment based on regular biomarker analysis. Its portable nature makes it suitable for point-of-care testing, providing immediate diagnostic results without requiring specialized laboratory equipment.

In terms of efficiency, an embodiment may provide rapid detection and real-time analysis, quicker than traditional methods like enzyme-linked immunosorbent assays (ELISA) or polymerase chain reaction (PCR), which involve multiple steps and longer processing times. An embodiment with compact and portable design may allow for point-of-care testing and immediate results, whereas conventional methods typically require specialized laboratory equipment and infrastructure. Regarding accuracy, an embodiment may show great sensitivity and can detect low concentrations of biomarkers, often achieving greater sensitivity than traditional methods such as immunoassays. Additionally, they offer label-free detection, which reduces errors associated with additional labeling and reagent steps, potentially enhancing accuracy. Therefore, in an embodiment, the analysis of urine samples involves minimal to no preparation of samples.

In an embodiment, the method of preparation of the disclosed biosensing device comprises dilution, correction of pH, and/or change of temperature, when the urine sample is not collected properly, including contamination, or otherwise is extremely abnormal regarding its physico-chemical properties.

An embodiment may comprise a method that combines classical signal processing techniques with advanced machine-learning models to clean noise from signals obtained by said device. The method aims to significantly improve the signal-to-noise ratio (SNR), reduce the mean squared error (MSE), and increase signal fidelity, thus ensuring more accurate and reliable detection of target molecules.

In an embodiment, the method for noise reduction improves SNR of transduced signals by integrating one or more classical signal processing techniques with one or more machine-learning models. These techniques may collectively work to identify and separate the actual biomarker signals from noise. The algorithm processes the acquired signal in stages, each designed to filter out different types of noise, such as high-frequency electrical noise, baseline drifts, and other artifacts. By enhancing the clarity of the true signal, the algorithm significantly boosts the SNR, leading to more accurate detection of biomarkers.

In an embodiment, the noise reduction method may comprise a Principal Component Analysis (PCA), Discrete Wavelet Transform (DWT), Kalman Filter, or a combination of these. In particular, the PCA reduces dimensionality by identifying and focusing on the principal components of the signal that account for most of the variance, effectively filtering out less significant noise components. Also, in particular, the DWT decomposes the signal into different frequency components, allowing for the isolation and removal of noise at specific frequencies without affecting the overall signal structure. Thirdly, in particular, the Kalman Filter estimates the true signal from noisy measurements by predicting the next state of the signal and correcting it based on the observed measurements, thus reducing the impact of random noise.

In an embodiment, the use of machine-learning models can enhance the effectiveness of the noise reduction method by enabling it to continuously learn from the signal data, identifying complex patterns of noise, and separating said patterns from the true biomarker signals with high precision. Machine-learning models can dynamically adjust to varying noise conditions and improve over time with continuous learning.

In an embodiment, the machine learning model may be selected from the following: Hybrid Deep Learning Model; Adaptive Noise Filtering using Reinforcement Learning (RL); Ensemble Learning with Boosting Techniques. The Hybrid Deep Learning Model may comprise a combination of Convolutional Neural Networks (CNNs) and Recurrent Neural Networks (RNNs) with Long Short-Term Memory (LSTM) units. CNNs are used for spatial feature extraction from the signal data, while RNN-LSTM units model the temporal dynamics of the signal, enhancing noise differentiation.

In an embodiment, ensemble learning and boosting are commonly used in predictive modeling but also be used for noise reduction, namely in biosensing devices. Utilizing ensemble methods to combine multiple noise reduction models into a single robust method through boosting techniques may be an interesting approach for biosensing.

In an embodiment, the Adaptive Noise Filtering using RL may be a useful reinforcement learning framework that continuously optimizes its noise filtering strategy based on feedback, adapting to real-time changes in noise levels and types. RL's dynamic adaptation capability may be suited for varying noise conditions in biosensing. RL, in the context of noise reduction for biosensors, may be a novel way of continuously improving noise filtering strategies, which is not commonly seen in traditional signal processing techniques. Implementing RL for adaptive filtering in biosensing devices may allow a shift from static filtering methods to a more dynamic and learning-based approach.

An embodiment to also reduce noise in transduced MOSFET measurements bat comprise the Ensemble Learning with Boosting Techniques such as Implementing Gradient Boosting Machines (GBM), or AdaBoost, to combine multiple noise reduction models, enhancing the overall robustness and accuracy by leveraging diverse model strengths.

Utilizing ensemble methods to combine multiple noise reduction models into a single robust model through boosting techniques is an important aspect of the present disclosure in the context of biosensing. Ensemble learning and boosting are commonly used in predictive modeling, but applying them to noise reduction in biosensing devices introduce may be of importance to exemplary embodiments. These approaches may not only improve the SNR but may also ensure the adaptability and robustness of the proposed biosensing device for detecting biomarkers.

An exemplary embodiment may use the disclosed method that differentiates between noise and actual biomarker signals by analyzing the signal characteristics and patterns using classical and/or machine-learning methods, and identifying patterns and anomalies that match known biomarkers and distinguish irregular and random variations as noise.

In an embodiment, the parameters used to identify and filter out noise comprise frequency content for isolating frequency bands typical of noise, temporal consistency for recognizing stable signal patterns associated with biomarkers, and/or statistical properties for identifying deviations from statistical norms.

In an embodiment, the noise reduction method can adapt to different levels or types of noise in urine samples. For these cases, the algorithm adjusts its processing methods via and Adaptive Filtering for continuously updating filter coefficients to minimize noise; and/or machine-learning for utilizing trained models to dynamically respond to new noise patterns.

Training data is collected through controlled experiments where biomarkers are spiked into urine samples. It is validated by comparing the algorithm's output against known biomarker concentrations and ensuring consistency across multiple tests. This data includes signals from urine samples with known concentrations of biomarkers, along with various noise profiles.

The advanced noise reduction method shows superior performance, compared to traditional methods, by providing higher SNR and more accurate detection of biomarkers.

In an embodiment, the results of the detection of biomarkers in urine samples using the biosensing device comprise an increased SNR by a factor of 2-3 times, when compared to traditional methods; an improved detection accuracy, greater than 90%, in detecting specific biomarkers, and a lower limits of detection for specific biomarkers.

In an embodiment, the computational requirements may include a moderate-level processor with sufficient memory to handle real-time data processing and machine-learning model inference.

In an embodiment, the algorithm may operate in real-time or near real-time conditions, to facilitate timely detection and analysis.

An embodiment comprises the disclosed algorithm that may be tested and validated through extensive laboratory experiments and field tests, with actual urine samples containing known biomarker concentrations. Furthermore, in an embodiment, to evaluate the performance of the device, the metrics and/or benchmarks comprise: Signal-to-Noise Ratio (SNR); Detection Accuracy; False Positive/Negative Rates; Processing Time; or combinations thereof.

In an embodiment, the noise reduction method may be customized or tuned for specific applications of the biosensing device, in particular, filter coefficients, for adaptive filtering techniques; model parameters, mor machine-learning models based on the type and level of noise; and threshold levels, for detecting specific biomarkers based on application requirements.

Maintaining a healthy balance of urine pH and metabolite concentrations is crucial for detecting potential health issues like urinary tract infection, kidney failure, and metabolic disorders. To address this, the present disclosure describes a biosensor based on field-effect transistors. This disclosed device comprises a microfabricated gold electrode to, not only detect urine pH, but also specific biomarkers. As a pH sensor, the present disclosure may show a good response, with a corresponding sensitivity of 2.20 µA/pH. For biomarker detection, the device successfully detected Immunoglobulin G, with antigen-antibody binding causing a measurable change of approximately 4 µA in its response.

In this way, one objective of the present disclosure is to describe a biosensor device based on FET, also referred to as Bio-FET device, capable of measuring urine pH and multiple urine metabolites.

The following pertains to materials and methods. In an embodiment, two components were considered for said biodevice: a custom-designed microfabricated gold electrode, working as an extended-gate (EG), and also referred as a chip, which is functionalized and exposed to the target analyte; and a readout circuit responsible for recording and amplifying signals resulting from modifications in the gate's electrical properties.

In an embodiment, none of the existing solutions has a retracted baseboard, so as to allow the incorporation of an "elevation support" embedded in the device itself.

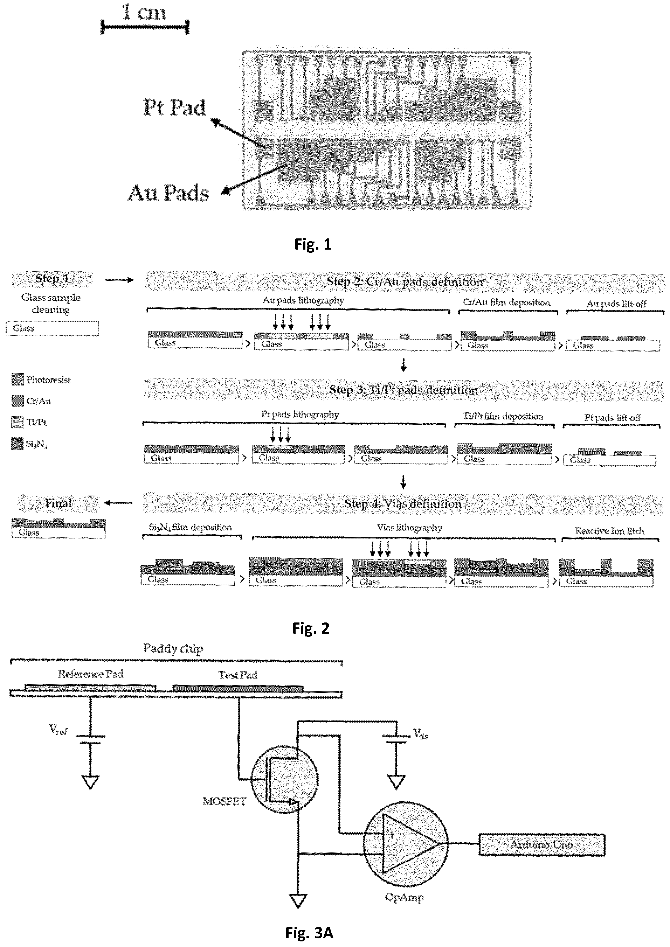

The following pertains to the chip microfabrication. In an embodiment, to test the optimal dimensions and forms of the gold EG, the chip, represented in **Figure 1****,** was microfabricated at a cleanroom facility. This chip comprises a total of 16 pads with different sizes and aspect ratios (length:width) divided into two different groups: the test pads; and the reference pads. The latter group was comprised of two platinum pads located in the first and last positions of the sequence of pads, among which are the remaining 14 gold test pads. Said pads may have a range of length and/or width dimensions from 0.1 mm to 4.5 mm, as seen in **Table 1.**

**Table 1: Dimensions of the test and reference pads of the embodiment of Figure 1.**

| **Pad** | **Dimensions** | **Pad** | **Dimensions** | **Pad** | **Dimensions** | **Pad** | **Dimensions** |
|---|---|---|---|---|---|---|---|
| **REF1** | **5 mm²** (2.25 x 2.25) mm² | **#4** | **2 mm²** (1.4 x 1.4) mm² | **#8** | **0.1 mm²** (0.325 x 0.325) mm² | **#12** | **0.5 mm²** (1.0 x 0.5) mm² |
| **#1** | **20 mm²** (4.5 x 4.5) mm² | **#5** | **1 mm²** (1.0 x 1.0) mm² | **#9** | **10 mm²** (4.5 x 2.25) mm² | **#13** | **0.2 mm²** (1.0 x 0.2) mm² |
| **#2** | **10 mm²** (3.25 x 3.25) mm² | **#6** | **0.5 mm²** (0.7 x 0.7) mm² | **#10** | **4.5 mm²** (4.5 x 1.0) mm² | **#14** | **0.1 mm²** (1.0 x 0.1) mm² |
| **#3** | **5 mm²** (2.25 x 2.25) mm² | **#7** | **0.2 mm²** (0.45 x 0.45) mm² | **#11** | **4.5 mm²** (3.25 x 1.4) mm² | **REF2** | **5 mm²** (2.25 x 2.25) mm² |

In an embodiment, the microfabrication process, illustrated in **Figure 2****,** was adapted from the one reported by Baldacchini et al. [4]. The manufacturing process was carried out in a glass substrate previously cleaned in an Alconox solution in an ultrasonic bath for 45 minutes, and then rinsed with isopropyl alcohol (IPA), deionized (DI) water and blow dried with nitrogen. Subsequently, three lithography steps were performed. The first lithography step defined the test electrodes. Herein, a 1.5 µL layer of a positive photoresist (e.g JSR Micro PFR 7790G-27cP) was spin-coated onto the sample. This photoresist was then exposed using a 422 nm wavelength laser in a direct write laser machine (DWL, Heidelberg Instruments). Completed the exposure process and in order to obtain a soft mask containing the sensor patterns, the sample as dipped into a suitable developer (JSR Micro PTH70EG solvent). Then through a magnetron sputtering machine (Alcatel SCM 450), a Cr/Au thin film (5 nm/200 nm) was deposited followed by lift-off to remove the Cr/Au from the undesired locations on the top of the mask, obtaining thus the required pattern. A second lithography step following the same recipe was performed before the deposition of a Ti/Pt thin film (5 nm/300 nm) to define the reference electrodes. The deposition is also done in the Alcatel SCM 450 machine. After the fabrication of all electrodes, the sample was fully passivated by depositing 400 nm of Si₃N₄ with a plasma-enhanced chemical vapor deposition (PECVD) machine (Oxford Plasma Pro 100 PECVD). Then the final lithography step was done to open contact vias on the nitride, leaving the pads exposed for later biofunctionalization.

In an embodiment, a second chip was also microfabricated at a cleanroom facility. The main distinction between this chip and the initial one is that the reference pads on the second chip are made of gold instead of platinum. Consequently, a second microfabrication process was performed using the same procedure as before but omitting the step for defining the platinum reference pads.

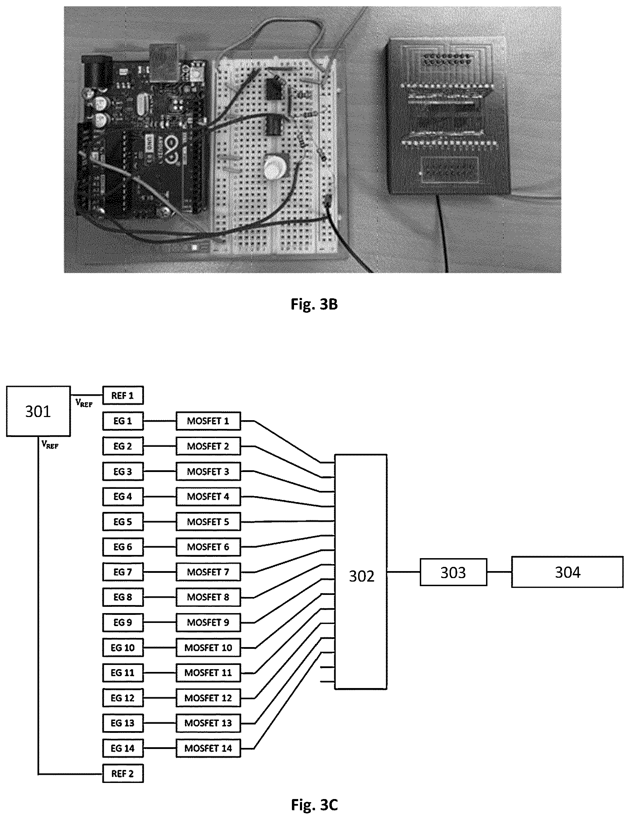

The following pertains to microelectronic circuit. In an embodiment, when the biorecognition elements on the gate electrode interact with the target biomolecules, they trigger a quantifiable modification in the gate's electrical properties of the extended gate, using a proper microelectronic circuit. The microelectronic circuit, illustrated in **Figure 3A****,** and photographically shown in a bread-board in **Figure 3B****,** consists of a sensing surface (chip) and the transducer element, the MOSFET, coupled with a readout circuit comprised of an operational amplifier to amplify the drain-source current (I_{ds}) and an Arduino to process such transducer signals using at least one signal processing method and/or at least one machine learning method.

**Figure 3C** illustrates the circuit for processing signals from pads of different sizes, where each EG/pad is connected to the gate terminal of an individual MOSFET, where: **301** represents a voltage source; **302** represents a multiplexer; **303** represents an amplifier, in particular, an operational amplifier; and **304** represents a data processor, in particular an Arduino, a Raspberry Pi or a microcontroller.

In an embodiment represented in **Figure 3C****,** each EG is responsible for detecting a specific biomarker. For sequential acquisition of signals from the various EGs, a multiplexer **302** controlled by a microcontroller **304** will be used. Additionally, an operational amplifier **303** will send the amplified signals to the microcontroller **304** for signal acquisition and processing. This configuration allows for sequential and efficient detection of biomarkers.

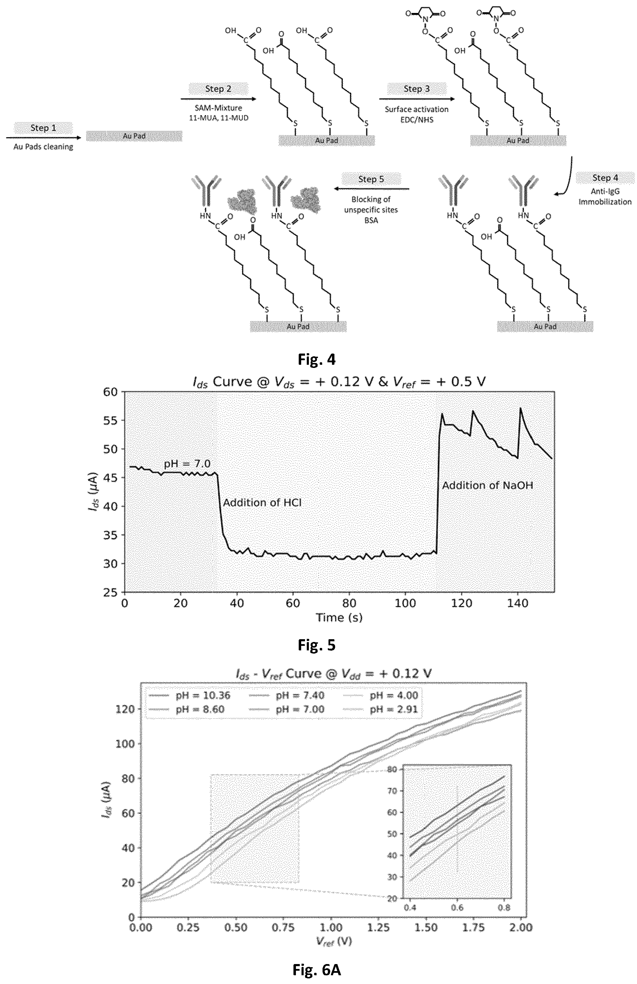

The following pertains to Surface Functionalization. In an embodiment, the functionalization of the precleaned chip was realized by a multistep biofunctionalization process given in **Figure 4****.** Prior to functionalization, the sample was cleaned with DI water and ethanol, then treated with ultraviolet (UV) light for 30 minutes to eliminate any contaminants from the gold surfaces. The first step was to incubate the EG gold pads, overnight at room temperature, in an ethanolic solution of mercaptoundecanoic acid (MUA) and 11-mercapto-1-undecanol (MUD), both purchased from Aldrich Chem. Co., at a concentration ratio of 0.33 mM to 0.67 mM (1:2) respectively, to form a mixed self-assembly monolayer (SAM). Then, to remove unbound molecules, the EG was successively rinsed with ethanol and DI water and dried with pure nitrogen. The activation of the carboxyl groups of the deposited SAM was achieved by incubating the sensor for 60 minutes in a solution of N-ethyl-N'-(3-(dimethylamino)propyl) carbodiimide (EDC)/N-hydroxysuccinimide (NHS), both purchased from Sigma-Aldrich, at 0.4 M/0.1 M ratio in Millipore water. After incubation, the surface was rinsed with Millipore water and blow-dried with nitrogen. Then, the EG was incubated overnight at 4 °C, with anti-immunoglobulin G (Anti-IgG) (Sigma-Aldrich) at a concentration of 0.01 mg/mL followed by rinsing the sensor with DI water and blow drying with nitrogen. Finally, to avoid nonspecific binding, the unbound sites on the electrode surface were blocked by incubation with a 1 mg/mL bovine serum albumin (BSA) (Sigma-Aldrich) solution in phosphate-buffered saline (PBS) 1X solution at pH 7.4 for 60 minutes at room temperature.

The following pertains to sensor regeneration. In an embodiment, the process of regenerating the sensor surface, with the aim of removing the bond between the target analyte and the immobilized recognition element, comprises two main steps: treatment with the regeneration solution and final washing with buffer solution to restore the initial conditions of the sensor. A possible approach to breaking the bond between the analyte and the receptor is based on overcoming their binding affinity. Although the underlying mechanism - the breakdown of non-covalent interactions - is common, the methods for achieving this can vary, including, among others, the application of light, temperature or changes in the chemical environment.

In an embodiment, heat treatment is used, namely the application of heated water at a temperature close to the melting temperature of the biomarker, as a way to promote the dissociation of the analyte-receptor complex. Considering this, water heated to 60 °C was used as the regeneration solution.

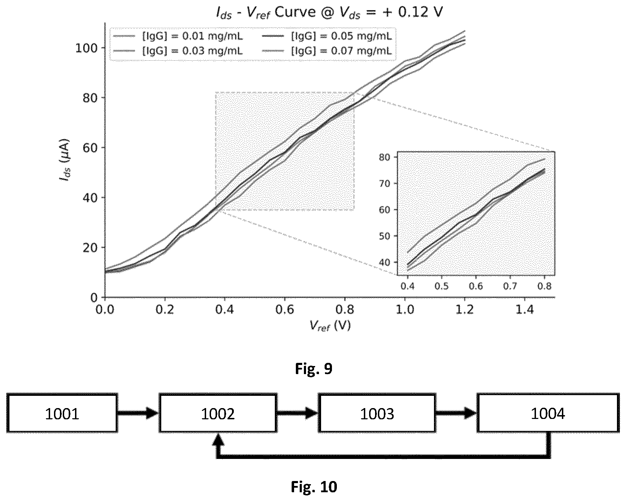

**Figure 10** shows an embodiment of the method for the regeneration tests. The process involves four sequential steps: **1001** represents the measurement of the baseline signal in buffer solution, in this case PBS, **1002** represents the detection of the biomarker signal, **1003** represents regeneration of the sensor surface with water at 60 °C, and **1004** represents a confirmation of sensor reset with a new measurement in PBS. The cycle is then repeated.

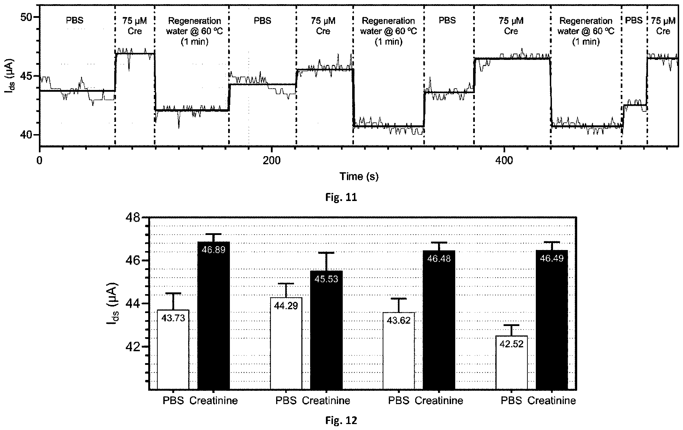

In an embodiment, the response of the biofunctionalized sensor was evaluated by real-time monitoring of the I_{ds} value during cyclic exposures to PBS and creatinine solutions, interspersed with regeneration steps using water for 1 minute, as represented in **Figure 11****.** For a quantitative analysis of the sensory response, the mean I_{ds} values for each condition (PBS and creatinine) were extracted directly from the real-time acquisition shown in **Figure 11****.** These values are represented graphically in **Figure 12****,** allowing a clear comparison between the different phases of each cycle.

Surprisingly, the results shown in **Figures 11 and 12** show that the introduction of creatinine into the system resulted in an immediate and consistent increase in I_{ds}, indicating a specific interaction between creatinine and the biorecognition element immobilized on the surface of the extended gate. After each detection cycle, thermal regeneration allowed the recovery of the baseline current associated with PBS, demonstrating effective regeneration of the sensor surface. This behaviour was consistently reproduced over four consecutive cycles, demonstrating the good reversibility and reusability of the sensor.

The following pertains to ellipsometric measurements. In an embodiment, conventional ellipsometry is utilized to measure optical parameters and estimate the thickness of each layer. Measurements were performed in air at a 70° angle of incidence using a SE 400 Ellipsometer (Sentech Instruments) equipped with a He-Ne laser with a wavelength of 632.8 nm. A two-phase model (gold/air) is employed to determine the refractive index (ns) and adsorption coefficient (ks) of the EG surface. For layer thickness measurement on the gold surface, a three-phase model (gold/film/air) was applied. Assuming a constant refractive index (nu) of 1.480 [5] for each layer, thickness (Tu) and extinction coefficient (ku) were determined through data fitting.

The following pertains to Biosensing Experiments and pH Tests. In an embodiment, to evaluate the functionality of the readout circuit and the chip, pH tests were conducted in two sets of experiments. The first aimed to study, in real-time, how the biosensing device reacts to changes in the solution pH, while the second group aimed to assess the sensitivity of the device to pH variations.

Regarding the first group of tests, to study the real-time response of the biosensing device to variations in solution pH, the source-drain current (I_{ds}) was measured. Herein, platinum and gold pads of the Paddy chip were used as reference and test pads, respectively. A buffer solution of pH 7 was applied onto the pads surface and constant voltages, both between drain and source terminals (V_{ds}=0.12 V) and at the gate electrode (V_{ref}=0.5 V) were applied. Then, hydrochloric acid (HCl) (1 M, Sigma-Aldrich) and sodium hydroxide (NaOH) (1 M, Sigma-Aldrich) were sequentially added to the buffer solution to induce pH changes. The current over time curve was obtained.

In the second set of experiments, the same pair of pads was used and six solutions with different pH values (10.36, 8.60, 7.40, 7.00, 4.00 and 2.91) were tested. The solutions were delivered to the pads using a micropipette. Herein, an electrical potential difference applied to the gate was scanned over a range of 0 to 2 V, maintaining a constant potential between the source and the drain (V_{ds}=0.12 V). The current-voltage (I_{ds}-V_{ref}) curves for each solution was obtained and compared. Then, a gate voltage value was selected within the linear operating region of the MOSFET, and for each pH value the respective I_{ds} value was recorded, allowing for the determination of the calibration curve (I_{ds}-pH).

The following pertains to Immunoglobulin G Tests. In an embodiment, to study the real-time response of the biosensing device to the presence of immunoglobulin G (IgG) (Sigma-Aldrich) and to evaluate the biosensor surface regeneration process, PBS was initially applied to the pads of the chip, followed by the addition of IgG to observe the system's response of the biosensing device, namely the I_{ds} value. Subsequently, Glycine-HCl was introduced to the disclosed device to regenerate the biosensor's surface. Herein, constant voltages, both between drain and source terminals (V_{ds}=0.12 V) and at the gate electrode (V_{ref}=0.7 V) were applied and the current over time curve was obtained.

In an embodiment, to assess the limit-of-detection (LOD), linear range, and sensitivity of the biosensing device, PBS solutions spiked with IgG, at different concentrations (0.01, 0.03, 0.05 and 0.07 mg/mL) were placed onto the pads using a micropipette and tested. For a constant drain-source voltage (V_{ds}=0.12 V), the gate voltage value was ranging from 0 to 1.2 V. The current-voltage curve (I_{ds}-V_{ref}) for each PBS-lgG solution was obtained.

The following pertains to Results and Discussion and pH Tests. Regarding the first set of tests, the changes in the drain-source current over time are represented in the plot of **Figure 5****.** In an embodiment, the results demonstrated a clear correlation between solution pH and drain-source current. Adding the acidic solution (hydrochloric acid) caused a decrease in current, while the sodium hydroxide solution led to an increase. This indicates that higher pH values lead to higher drain-source currents.

To assess the sensitivity of the biosensing device to pH variations, the current-voltage (I_{ds}-V_{ref}) curves of the six solutions tested were obtained and compared, as illustrated in **Figure 6A****.** The inset plot shows the response of the MOSFET when operating in the linear region (0.4 V to 0.8 V). Surprisingly, current-voltage curves of the biosensing device differed depending on the pH of the solution. Higher pH values of the substance in contact with the EG gave rise to higher drain-source current values.

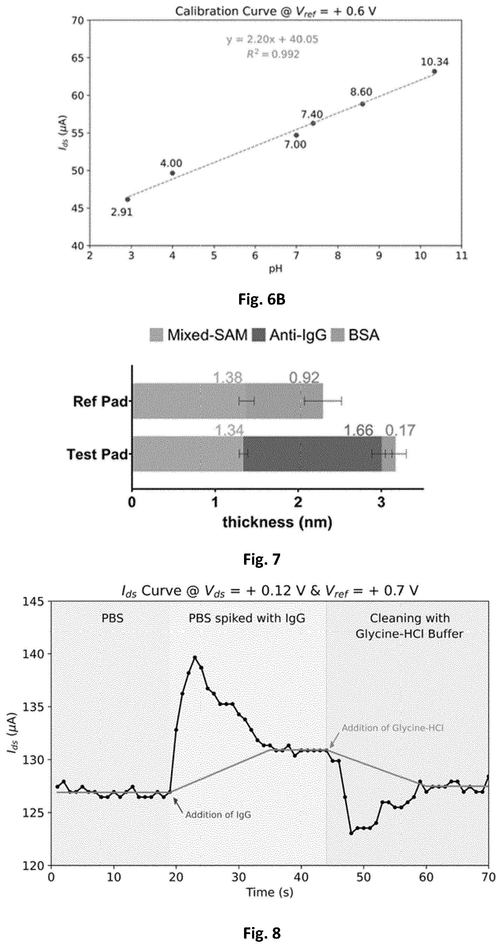

In an embodiment, the calibration curve of the disclosed device is obtained for each reference voltage value (V_{ref}) within the linear operating region of the MOSFET. In the case of **Figure 4A,** reference voltage of 0.60 V is selected for an embodiment, and is represented by a red vertical line in the inset plot of **Figure 6A****,** with the respective drain-source current value being recorded for each pH value. In **Figure 6B****,** a calibration curve I_{ds}-pH is shown to be linear, with a sensitivity of 2.20 µA/pH.

The following pertains to Ellipsometric Measurements. In an embodiment, each step of the functionalization process and consequent surface modification was characterized and validated by ellipsometry, so as to determine the thickness of each deposited layer. In **Figure 5****,** the thicknesses of each deposited layer for the pair of pads used in the IgG tests are illustrated.

The mixed-SAM thickness can be calculated by multiplying its number of C-C bonds by the C-C length of 0.154 nm and by sin(60°), to consider the C-C bond angle [6]. Since, both the components used in this mixed-SAM have the same number of C-C bonds, its thickness are equal to 1.34 nm. From **Figure 5****,** it is possible to see that the thickness of the Mixed-SAM of both the pads are quite similar and agree with the expected value of 1.34 nm.

In an embodiment, the plot in **Figure 7** demonstrates that incubating the EG with the antibody solution resulted in an increased film thickness on the test pad, indicating successful immobilization of the antibody on the EG surface.

Moreover, comparing the BSA layer thickness on both pads reveals that the reference pad has a thicker layer than the test pad, which can be attributed to the number of binding sites available to block. The BSA layer on the test pad is thin, indicating minimal non-specific binding sites to block. In contrast, the reference pad has a thicker BSA layer as it was not incubated with Anti-IgG, resulting in more blocked non-specific binding areas.

The following pertains to IgG Tests. In **Figure 8****,** the current over time curve obtained when to a PBS solution was added to the device first IgG and then Glycine-HCl is shown. The results showed that, where IgG was added to the solution, there was an increase of the I_{ds} value, showing that the binding event between the IgG and the immobilized biorecognition element, in this case, Anti-IgG, affects the drain-source current. Additionally, when Glycine-HCl was added to the device, the current value returned to initial value without IgG.

The current-voltage curves obtained for each PBS-lgG solution tested are shown in the **Figure 9****.** The results showed that current-voltage curves differed depending on the IgG concentration of the solution, with higher IgG concentration associated with higher current values.

As a pH sensor, the Bio-FET device shows a good response, demonstrating a linear relationship with a sensitivity of 2.20 µA/pH. Regarding IgG detection, the introduction of IgG enhances the response of the biosensing device, highlighting the sensitivity of said device to IgG presence. Additionally, Glycine-HCl has proven to be a promising cleaning solution, allowing for the regeneration of the sensor surface. At the same time, reusability shows that a number of times that the sensor surface can be regenerated without compromising the functionality of the biosensing device. Finally, the number of urinary biomarkers capable of being simultaneous detected by the disclosed biodevice can be straightforwardly expanded beyond IgG.

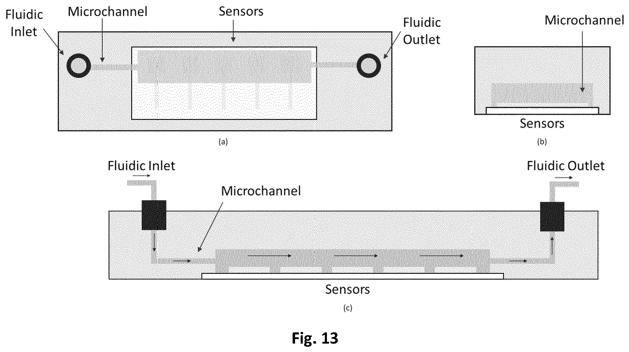

**Figure 13** schematically illustrates the structure and operation of an embodiment of the biosensing device designed for the analysis of multiple urinary biomarkers in a single sample. The diagram provides a detailed view of the flow within the channel, highlighting the fluid trajectory and the positioning of the different EGs. Urine enters through the inlet, travels through the microchannel, and exits through the outlet. This microchannel was developed to ensure uniform and controlled flow over the EG matrix, where each EG is responsible for detecting a specific biomarker.

The embodiments described are combinable with each other. The present invention is, of course, in no way restricted to the embodiments described in this document, and a person with average knowledge in the art will be able to predict many possibilities of modifying it and substituting technical features with equivalent ones, depending on the requirements of each situation, as defined in the attached claims. The following claims define additional embodiments of the present description.

### References

[1] Sequeira-Antunes, B.; Ferreira, H.A. Urinary Biomarkers and Point-of-Care Urinalysis Devices for Early Diagnosis and Management of Disease: A Review. Biomedicines 2023, 11, 1051.
[2] Masajtis-Zagajewska, A.; Nowicki, M. New markers of urinary tract infection. Clinica chimica acta 2017, 471, 286-291.
[3] Hwang, C.; Lee, W.-J.; Kim, S.D.; Park, S.; Kim, J.H. Recent Advances in Biosensor Technologies for Point-of-Care Urinalysis. Biosensors 2022, 12, 1020.
[4] Baldacchini, C.; Montanarella, A.F.; Francioso, L.; Signore, M.A.; Cannistraro, S.; Bizzarri, A.R. A reliable biofet immunosensor for detection of p53 tumour suppressor in physiological-like environment. Sensors 2020, 20, 6364.
[5] Arwin, H. Optical properties of thin layers of bovine serum albumin, γ-globulin, and hemoglobin. Applied spectroscopy 1986, 40, 313-318.
[6] Phan, H.T.; Bartelt-Hunt, S.; Rodenhausen, K.B.; Schubert, M.; Bartz, J.C. Investigation of bovine serum albumin (BSA) attachment onto self-assembled monolayers (SAMs) using combinatorial quartz crystal microbalance with dissipation (QCM-D) and spectroscopic ellipsometry (SE). PloS one 2015, 10, e0141282.

## Claims

1. Biosensing device for measuring at least one biomarker of a human biological sample, comprising a channel for allowing the flow of the human biological sample, an inlet at one end of said channel and an outlet the other end of said channel, wherein the said channel comprising:
a sensor for transducing a signal of the at least one biomarker of the biological sample;
an amplifier electrically connected to the sensor for amplifying the transduced signal; and a computer processor configured to:
acquire the amplified signal, and
quantify the at least one biomarker in the biological sample;
wherein the sensor comprises one or more field-effect transistors, FET;
wherein each of the one or more the field effect transistor, FET, comprises an extended gate; and
wherein each of the one or more extended gates comprises a plurality of analyte-binding elements for detecting the at least one biomarker.

2. Biosensing device according to the previous claim comprising a toilet or a urinal, wherein the inlet is connected to the toilet, or to the urinal for receiving the human biological sample, and wherein the outlet is connected to a sewer or to a sterilized container.

3. Biosensing device according to any of the previous claims, wherein the human biological sample is at least one of urine, blood, saliva, sweat and feces, in particular, the human biological sample is urine.

4. Biosensing device according to any of the previous claims comprising a glass or ceramic surface, wherein the sensor is arranged on the glass or ceramic surface, and wherein:
said glass or ceramic surface is suitable to be sterilized;
the one or more extended gates are placed onto said glass or ceramic surface; and
wherein each of the one or more extended gates comprises:
a chromium layer deposited onto said glass or ceramic surface; and
a gold layer placed deposited on the chromium layer, on which are arranged the plurality of analyte-binding elements.

5. Biosensing device according to the previous claim, comprising a direct-current voltage source, wherein the sensor comprises one or two reference electrodes [i.e., reference pads], wherein said reference electrodes are electrically connected to the direct-current voltage source, and wherein each of the one or two reference electrodes comprises:
a titanium layer; and
a platinum layer deposited on the titanium layer, for electrically connecting to the direct current voltage source.

6. Biosensing device according to the previous claim, wherein the titanium layer of at least one of the one or two reference electrodes is deposited on the gold layer, and wherein the gold layer is deposited onto the chromium layer, and further wherein the chromium layer is deposited onto the glass or ceramic surface [typical thicknesses of the layers are as follows: electrodes with 5 nm of chromium and 200 nm of gold; electrodes with 5 nm of titanium and 300 nm of platinum].

7. Biosensing device according to claim 5 or 6, wherein the direct-current voltage source is from 0.2 V to 2 V; more preferably from 0.4 V to 0.8 V.

8. Biosensing device according to any of the previous claims, wherein the sensor further comprises a multiplexer for multiplexing each current signal from each of the one or more field-effect transistors, wherein each of the two or more field-effect transistors is electrically connected to the multiplexer.

9. Biosensing device according to any of the previous claims, wherein each of the one or more extended gates has a length ranging from 0.1 mm to 10 mm; preferably from 0.5 mm to 5 mm; and/or wherein the extended gate has an aspect ratio of width per length ranging from 1:100 to 1:1; preferably from 1:10 to 1:1.

10. Biosensing device according to any of the previous claims, wherein acquiring the amplified signal comprises a step of removing a noise component from said demultiplexed signal.

11. Biosensing device according to any of the previous claims comprising a revolving assembly of a plurality of sensors, wherein each sensor of the plurality of sensors comprises one or more field-effect transistors, FET, wherein each of the one or more the field effect transistor, FET, comprises an extended gate, and wherein each of the one or more extended gates comprises a plurality of analyte-binding elements for detecting the at least one biomarker.

12. Method for obtaining a biosensing device according to any of the previous claims, comprising the following steps for obtaining the one or more extended gates and the one or two reference electrodes:
providing a glass surface;
coating the glass surface with a layer of a first photoresistor material;
patterning onto the first photoresistor layer by laser direct-writing;
depositing a chromium layer and a gold layer onto the glass surface and the patterned first photoresistor layer;
removing the first photoresistor layer to obtain the glass surface with patterned chromium and gold layers;
coating with a layer of a second photoresistor material;
patterning onto the second photoresistor layer by laser direct-writing;
depositing a titanium layer and a platinum layer onto the onto the second photoresist and the patterned chromium and gold layers;
removing the second photoresistor layer to obtain patterned titanium and platinum layers.

13. Method according to the previous claim, further comprising the steps of:
incubating the one or more extended gates with an ethanolic solution of mercaptoundecanoic acid, MUA, and 11-mercapto-1-undecanol, MUD, with a molar proportion ranging from 1:3 to 2:3 (%mol._{MUA}/%mol._{MUD}), for the formation of a mixed self-assembly monolayer (SAM);
incubating the extended gate with a solution of the analyte-binding elements;
incubating the extended gate with a bovine serum albumin, BSA, solution, for blocking unbound parts of the extended gate.

14. Method for measuring biomarkers of a biological sample, comprising the steps:
feeding the biosensing device according to any of the previous claims with a biological sample; and
adding a Glycine-HCl regeneration flow to the one or more extended gates, after operation of the sensor, in particular after each operation of the sensor.

15. Method for measuring biomarkers of a biological sample according to the previous claim, wherein said biomarkers includes a pH biomarker.
